Europäisches Patentamt

(19) European Patent Office

Office européen des brevets

(11) Publication number: **0 119 852**
**B1**

(12) **EUROPEAN PATENT SPECIFICATION**

(45) Date of publication of patent specification: 29.07.87          (51) Int. Cl.⁴: **A 61 K 31/365**

(21) Application number: 84301813.6

(22) Date of filing: 16.03.84

(54) **Podophyllotoxin preparations for use in the treatment of genital warts.**

(30) Priority: 18.03.83 GB 8307614

(43) Date of publication of application:
26.09.84 Bulletin 84/39

(45) Publication of the grant of the patent:
29.07.87 Bulletin 87/31

(84) Designated Contracting States:
AT BE CH DE FR GB IT LI LU NL SE

(56) References cited:
GB-A-1 145 909

(73) Proprietor: **Pharma-medica a-s**
**19 Vesterlundevj**
**DK-2730 Herlev (DK)**

(72) Inventor: **Jacobsen, Mette Kirstine**
**Lojtegardsvej 145**
**DK-2770 Kastrup (DK)**
Inventor: **Malmstedt, Marianne**
**Enghojvej 21 Ganlose**
**DK-2760 Malov (DK)**

(74) Representative: **Ritter, Stephen David et al**
**Mathys & Squire 10 Fleet Street**
**London EC4Y 1AY (GB)**

EP 0 119 852 B1

Courier Press, Leamington Spa, England.

## Description

This invention relates to pharmaceutical compositions for the treatment of condylomata acuminata.

Condylomata acuminata or genital warts is a sexually transmitted disease which affects both males and females. In their clinical appearance fresh condylomata appear as greyish-pink pedunculated papillomatous vegetations while older tumors may gradually turn into erythematous or pigmented papules. In non-circumcised men, the preputial cavity is most often diseased, while in the circumcised male it is normally the shaft of the penis which is affected. In females, clinically evident lesions predominantly grow in the vulvavestibular area, although the vagina is also often afflicted.

Treatments hitherto have included tedious and frequently unsuccessful surgical measures and chemotherapy. The first choice medical treatment since about 1950 has been application of an ethanolic solution of podophyllin resin. Podophyllin resin is obtained from the root extract of Podophyllum peltatum or Podophyllum emodi or hexandrum and can contain one or more of the following active ingredients: podophyllotoxin, 4'-demethylpodophyllotoxin, α-peltatin, and β-peltatin.

The use of ethanolic solutions of podophyllin has a number of disadvantages. Firstly, the efficacy of such solutions may be limited, secondly, the actual effect of the treatment may be difficult to reproduce, thirdly the presence of components of podophyllin resin other than podophyllotoxin in variable quantities can affect the safety of the treatment and fourthly, previous formulations had a limited shelf-life. Also neither the podophyllin resin nor the ethanolic solution can be readily standardized biologically or chemically. Furthermore the previous preparations are highly irritant to the skin and mucous membranes, which has led to a general treatment regime including washing off the drug 2—6 hours after application. Irritation will arise not only in the condylomata as they start becoming necrotic as a result of the antimitotic effect of the podophyllin but also in the adjacent healthy skin or mucosa. It is therefore of crucial importance that the preparations are applied precisely onto the condylomata. This is, however, only possible to some degree as e.g. on penile warts. When treating female patients it is a very difficult task to carry out precise application of the drug to the condylomata only, as they are located internally. The same holds good for anal warts.

For that reason, treatment of condylomata has hitherto mainly been performed in clinics or hospitals, which is a rather expensive method of treatment.

Another disadvantage is that absorption of the active ingredient into the condylomata may be erratic because the preparation may be wiped off by contact with the clothes or may be rinsed off during micturition.

We now have discovered that a superior therapeutic composition for treatment of condylomata may be obtained by incorporation of 0.05—10% by weight of podophyllotoxin into a pharmaceutically acceptable carrier comprising at least one glycol selected from alkylene glycols and polyalkylene glycols. Preferably the composition has a viscosity and/or water solubility characteristics such that localised application of the composition may be achieved and displacement of the composition inhibited. Examples of suitable compositions include ointments, gels, particularly aqueous gels and flexible collodion vehicles. Compositions according to the invention have been found (1) to have an enhanced therapeutic effect, possibly due to an increase in local absorption rate of the active ingredient caused by the presence of the glycol, and (2) to be stable.

A suitable alkylene glycol is, for example, propylene glycol and a suitable polyalkylene glycol is, for example, polyethylene glycol. Preferably, compositions according to the invention comprise at least one polyethylene glycol having a molecular weight in the range from 100 to 1000, most preferably 200 to 600.

Advantageously, the compositions according to the invention are in the form of aqueous gels containing propylene glycol or polyethylene glycol 400 as solvent for the podophyllotoxin and at least one nonionic emulsifier and/or at least one surfactant. Alternatively the compositions may be in the form of lipid-based ointments which may, for example be based on white soft paraffin, beeswax, stearyl alcohol, or mineral oil. Use of compositions according to the invention, particularly those having lipophilic characteristics and a consistency such that they can be readily and accurately applied to the condylomata by the patient himself, can considerably reduce the risk of adverse effects to the surrounding areas. Lipophilic compositions could not have been safely used as vehicles for podophyllin in the treatment of condylomata as the risk of adverse effects would have been increased.

As indicated, the compositions of the invention can contain a lipid phase consisting of white soft paraffin, beeswax, stearyl alcohol mineral oil, or similar ingredients. The compositions (including lipid-based and non-lipid based such as aqueous gels) may also include surfactants (preferably non-ionic) such as Tagat® S, Hyfatol 40 and Tween® 80 and emulsifiers (also preferably non-ionic) as e.g. Emulgator E 2155 and Arlacel® 83.

The composition of the invention are preferably buffered to a pH less than 7, most preferably a pH in the range 2.5 to 6.0. By selecting the pH in this range the efficacy of the compositions of the inventions can be increased and furthermore the stability of the compositions is improved. The required buffering action may be obtained by incorporating a pharmacologically acceptable acid in the composition, for example lactic, phosphoric, malic or citric acids and/or pharmaceutically acceptable salts thereof. Thus, for example a lactic acid/lactate buffer of pH around 4.5 may be included.

2

**0 119 852**

As indicated, the compositions of the invention are stable and they can be chemically standardized by HPLC-assay method.

The compositions according to the invention are preferably produced by first dissolving podophyllotoxin in the glycol and then admixing the resulting solutions with the other ingredients of the compositions.

The following formulations according to the invention are given by way of example.

1. Formulation P.O. 2201

| Active ingredient | Podophyllotoxin | 5 g |
|---|---|---|
| Inactive ingredients | white soft paraffin | 596 g |
| | liquid paraffin | 159 g |
| | Tagat® S | 40 g |
| | Polyethylene glycol | 200 g |
| | | 1000 g |

The white soft paraffin, liquid paraffin and Tagat S® were melted and stirred together to form a first mixture and the podophyllotoxin was separately dissolved in polyethylene glycol. The two components were then mixed and the mixture stirred while cooling.

2. Formulation P.O. 2203

| Active ingredient | Podophyllotoxin | 5 g |
|---|---|---|
| Inactive ingredients | white soft paraffin | 230 g |
| | light liquid paraffin | 150 g |
| | Tagat® S | 100 g |
| | Hyfatol® 40 | 195 g |
| | Polyethylene glycol 400 | 200 g |
| | | 1000 g |

The white soft paraffin, light liquid paraffin, Tagat® S and Hyfatol® 40 were melted and stirred together to form a first mixture and the podophyllotoxin was dissolved in the polyethylene glycol 400. The two components were then mixed and the mixture stirred while cooling.

3. Formulation P.O. 3101 (A) and (B)

(A)

| Active ingredient | Podophyllotoxin | 5 g |
|---|---|---|
| Inactive ingredients | Emulgator E 2155 | 70 g |
| | Purified water | 95 g |
| | Avicel® | 60 g |
| | Polyethylene glycol 400 | 50 g |
| | Buffer | 20 g |
| | | 1000 g |

3

**0 119 852**

(B)

| Active ingredient | Podophyllotoxin | 5 g |
|---|---|---|
| Inactive ingredients | Emulgator E 2155 | 95 g |
| | Purified water | 657.5 g |
| | Avicel® | 80 g |
| | Polyethylene glycol 400 | 120 g |
| | Buffer (lactate pH 4.5) | 40 g |
| | Methyl paraben | 2 g |
| | Propyl paraben | 0.5 |
| | | 1000 g |

The water was heated to 80°C and the Emulgator E 2155 was dissolved while stirring. The solution was then cooled to 60°C. The Avicel® was then added while stirring and then the buffer and the methyl and propyl parabens (formulation B). The podophyllotoxin was then separately dissolved in the polyethylene glycol 400. The two components were mixed then stirred and the total mixture homogenised.

4—7. Formulations P.O. 3102, P.O. 3103, P.O. 3104 and P.O. 3105

| Active ingredient | Podophyllotoxin | 5 g |
|---|---|---|
| Inactive ingredients | white soft paraffin | 745 g |
| | Tween® 81 | 50 g |
| | Propylene glycol | 200 g |
| | | 1000 g |

| Active ingredient | Podophyllotoxin | 5 g |
|---|---|---|
| Inactive ingredients | white soft paraffin | 550 g |
| | white beeswax | 70 g |
| | Mineral oil | 135 g |
| | Propylene glycol | 150 g |
| | Arlacel® 83 | 90 g |
| | | 1000 g |

4

| Active ingredient | Podophyllotoxin | 5 g |
|---|---|---|
| Inactive ingredients | white soft paraffin | 687 g |
| | stearyl alcohol | 30 g |
| | white beeswax | 78 g |
| | Polyethylene glycol | 200 g |
| | | 1000 g |

| Active ingredient | Podophyllotoxin | 5 g |
|---|---|---|
| Inactive ingredients | white soft paraffin | 615 g |
| | white beeswax | 80 g |
| | Mineral oil | 200 g |
| | Polyethylene glycol | 100 g |
| | | 1000 g |

For each of the above formulations first the podophyllotoxin was dissolved in the propylene glycol or polyethylene glycol. Then the remaining ingredients were melted and mixed thoroughly and finally the two mixtures were combined and the combination stirred while cooling.

Stability tests carried out on the above described compositions revealed that formulations PO-2201, PO-2203, PO-3102, PO-3104 and PO-3105 were stable for only six months. Compositions PO-3105(A) and (B) appeared to be completely stable and were consequently preferred where long shelf-life is required. It is presently believed that the enhanced stability of compositions PO-3105(A) and (B) is due to the presence of the buffer which maintains the pH of the composition in the range of 2.5 to 6.0.

## Claims

1. A pharmaceutical composition suitable for treating genital warts comprising from 0.05 to 10% by weight of podophyllotoxin and a pharmaceutically acceptable excipient comprising at least one glycol selected from alkylene glycols and polyalkylene glycols.

2. A composition according to Claim 1 in the form of an ointment or gel.

3. A composition according to Claim 1 or Claim 2 wherein said excipient comprises propylene glycol.

4. A composition according to any preceding claim wherein said excipient comprises at least one polyalkylene glycol.

5. A composition according to Claim 4 wherein said excipient comprises polyethylene glycol having a molecular weight in the range from 100 to 1000.

6. A composition according to any preceding claim in the form of a lipid-based ointment.

7. A composition according to any of Claims 1 to 5 in the form of an aqueous gel.

8. A composition according to any preceding claim including buffering agents maintaining the pH of the composition in the range 2.5 to 6.0.

9. A process for producing a composition as claimed in any preceding claim which comprises dissolving the podophyllotoxin in the glycol and admixing the resulting solution with other components of the composition.

## Patentansprüche

1. Zur Behandlung von Genitalwarzen geeignetes Arzneimittel aus 0,05 bis 10 Gewichtsprozent Podophyllotoxin und einem pharmazeutisch verträglichen Träger, der mindestens ein Glykol umfaßt, das aus den Alkylenglykolen und Polyalkylenglykolen ausgewählt ist.

2. Mittel nach Anspruch 1, in der Form einer Salbe oder eines Gels.

3. Mittel nach Anspruch 1 oder 2, in dem der Träger Propylenglykol umfaßt.

4. Mittel nach einem der vorangehenden Ansprüche, in dem der Träger mindestens ein Polyalkylenglykol umfaßt.

5. Mittel nach Anspruch 4, in dem der Träger Polyäthylenglykol mit einem Molekulargewicht im Bereich von 100 bis 1000 umfaßt.

6. Mittel nach einem der vorangehenden Ansprüche in Form einer Salbe auf Lipidbasis.

7. Mittel nach einem der Ansprüche 1 bis 5 in Form eines wäßrigen Gels.

8. Mittel nach einem der vorangehenden Ansprüche, enthaltend Pufferstoffe, die den pH-Wert des Mittels im Bereich von 2,5 bis 6,0 halten.

9. Verfahren zur Herstellung eines Mittels nach einem der vorangehenden Ansprüche durch Auflösen des Podophyllotoxins in dem Glykol und Vermischen der erhaltenen Lösung mit den anderen Bestandteilen des Mittels.

**Revendications**

1. Une composition pharmaceutique convenant au traitement des végétations vénériennes comprenant de 0,05 à 10% en poids de podophyllotoxine et un excipient pharmaceutiquement acceptable comprenant au moins un glycol choisi parmi les alcoylèneglycols et les polyalcoylèneglycols.

2. Une composition selon la revendication 1 sous forme d'une pommade ou d'un gel.

3. Composition selon la revendication 1 ou la revendication 2 dans laquelle ledit excipient comprend du propylèneglycol.

4. Une composition selon l'une quelconque des revendications précédentes dans laquelle ledit excipient comprend au moins un polyalcoylèneglycol.

5. Une composition selon la revendication 4 dans laquelle ledit excipient comprend un polyéthylèneglycol ayant un poids moléculaire dans la gamme de 100 à 1 000.

6. Une composition selon l'une quelconque des revendications précédentes sous forme d'une pommade à base lipidique.

7. Une composition selon l'une quelconque des revendications 1 à 5 sous forme d'un gel aqueux.

8. Une composition selon l'une quelconque des revendications précédentes comprenant des agents tampons maintenant le pH de la composition dans la gamme de 2,5 à 6,0.

9. Un procédé pour la production d'une composition comme revendiqué dans l'une quelconque des revendications précédentes qui comprend la dissolution de la podophyllotoxine dans le glycol et le mélange de la solution obtenue avec les autres composants de la composition.